# EUROPEAN PATENT SPECIFICATION

(11) **EP 2 510 867 B1**
(45) Date of publication and mention of the grant of the patent: **10.01.2018**
(21) Application number: 12164022.1
(22) Date of filing: 13.04.2012
(51) Int. Cl.: A61B 1/005, A61B 1/00

(54) **ENDOSCOPE**
ENDOSKOP
ENDOSCOPE

(30) Priority: 14.04.2011 JP 2011090335
(43) Date of publication of application: 17.10.2012
(73) Proprietor: Fujifilm Corporation, Minato-ku Tokyo 106-8620 (JP)
(72) Inventor: KATSURA, Hirofumi, Ashigarakami-gun Kanagawa, 258-8538 (JP); OZAKI, Takao, Ashigarakami-gun Kanagawa, 258-8538 (JP); OBATA, Yoshihiro, Ashigarakami-gun Kanagawa, 258-8538 (JP); IYAMA, Shozo, Ashigarakami-gun Kanagawa, 258-8538 (JP); OHTA, Yasunori, Ashigarakami-gun Kanagawa, 258-8538 (JP); HOSONO, Yasuyuki, Ashigarakami-gun Kanagawa, 258-8538 (JP)
(74) Representative: Klunker IP Patentanwälte PartG mbB

(56) References cited:
- EP-A2- 2 074 927
- JP-A- H0 924 020
- JP-A- 2003 159 213
- US-A1- 2005 059 861
- US-B2- 6 860 849

## Description

### Field of the Invention

The present invention relates to an endoscope according to the preamble of claim 1. It also relates to a method of manufacturing an endoscope as defined by claim 1.

### Description of the Related Art

In the medical field, diagnoses and treatments using an endoscope have been widely performed and have wide-ranging use. For example, an image of the inside of a body cavity is shot by an image pickup device, such as a CCD, incorporated at the tip of an insertion part of an endoscope, and after various processes in a processor device, an image at a desired position and at a desired angle in the body cavity can be displayed on a monitor. Also, a treatment tool is inserted into a body cavity via a channel of the endoscope for insertion of a treatment tool, and a treatment such as polyp resection can be performed by the treatment tool.

In an insertion part of the endoscope to be inserted into a body cavity, a tip hard part provided with an objective lens or the like, a bending part (an angle part) flexing (bending) according to the operation by a user (an operator) via a hand operating part, and a soft part (a flexible part) bending freely along an insertion route are sequentially arranged from the tip side to the base end side. These tip hard part, bending part, and soft part configuring the insertion part have different functions and characteristics required, and have different internal structures according to the respective requirements. On the other hand, the bending part and the soft part have its outer circumference coated with an outer coat, thereby allowing the insertion part of the endoscope to be smoothly inserted into a body cavity.

In general, the bending part and the soft part have different structures and are therefore made as separate bodies. By bonding both the bending part and soft part together, the insertion part of an endoscope is made. In this case, by boding an outer coat of the bending part and an outer coat of the soft part together, an outer coat of the entire insertion part is formed.

The outer coat plays a role of reducing friction with respect to an internal organ at the time of insertion and making insertion and progression smooth while preventing body fluids and others from entering the endoscope (the insertion part) from outside. Therefore, it is required to bond the outer coat of the bending part and the outer coat of the soft part together so as to come into close contact with each other appropriately. In a configuration in which separate outer coats are bonded together, however, deterioration of the bonding part tends to proceed with repetition of use and cleaning, and much attention is required to be paid to vulnerability to chemicals, air leakage, and others at the bonding part.

Therefore, in view of providing an endoscope showing excellent fluid-tightness (air-tightness) with a simple structure over a longer period of time, the bending part and the soft part are preferably coated with an outer coat having an integral structure without a bonding part.

For example, Japanese Patent Application Publication No. 1-244733 discloses an endoscope where an outer-layer elastomer with which the outer circumference of a braid is coated forms an outermost layer of an outer coat in a bend tube part and a flexible tube. Also, Japanese Patent Application Publication No. 2001-137180 discloses an endoscope where a bending part and a soft part are coated with an outer coat tube.

When using an endoscope, a user is required to sequentially unreel an endoscope insertion part from any insertion port of a body into the body cavity to cause the insertion part to reach an affected area in the body cavity. In such a case, while checking a track image obtained by means of the objective lens at the tip hard part, the user controls a bending state of the bending part via the hand operating part and pushes, draws, or rotates the soft part on hand to ensure the track of the insertion part in the body cavity.

In this manner, the bending part and the soft part each have an appropriate level of stiffness (rigidity, hardness, or resilience) according to its role given thereto. For example, while the bending part is required to have a level of stiffness (flexibility) not hindering a bending operation, the soft part is required to have a level of stiffness at which the hand operation by the user can appropriately control even the tip of the soft part. In an endoscope with the outer coat of the bending part and the outer coat of the soft part being configured as separate bodies, by bonding separate outer coats together each having a level of stiffness required for each, an insertion part having an appropriate level of stiffness as a whole can be easily achieved.

However, when the bending part and the soft part are coated with an outer coat having an integral structure without a bonding part, it is difficult to provide the outer coat having an integral structure with an appropriate level of stiffness for each of the bending part and the soft part, and various contrivances are required.

In the endoscope disclosed in Japanese Patent Application Publication No. 1-244733, an inner-layer elastomer is provided as an inner layer of the outer coat. Also, in the endoscope disclosed in Japanese Patent Application Publication No. 2001-137180, a thin tube of the soft part is coated with an outer coat tube.

In the endoscope disclosed in Japanese Patent Application Publication No. 1-244733, however, the inner-layer elastomer with which only the flexible tube part is coated is arranged inside the outer-layer elastomer, and therefore it is not necessarily easy to perform relative positioning and fixing the positions of the inner-layer elastomer and the outer-layer elastomer at the time of manufacture. Similarly, in the endoscope disclosed in Japanese Patent Application Publication No. 2001-137180, the thin tube with which only the soft part is coated is arranged inside the outer coat tube, and it is not necessarily easy to perform relative positioning and fixing the positions of the thin tube and the outer tube.

EP 2 074 927 A discloses an endoscope having two different coats for the soft part and the bending part, respectively.

JP 2003 159 213 A and JP 9-24020 disclose endoscopes according to the preamble of claim 1. The first outer coat provided in the soft part may be made from a metal such as aluminum, while the first outer coat provided in the bending part is constituted by rubber material. To prevent explosion of such material under high internal pressure of the endoscope counter measurements are to be taken.

### SUMMARY OF THE INVENTION

The present invention has been contrived in view of these circumstances, and has an object of providing an endoscope in which different levels of stiffness required for a soft part and a bending part respectively can be easily obtained with high accuracy while excellent fluid-tightness can be ensured from the soft part to the bending part.

One aspect of the present invention is directed to an endoscope comprising the features of claim 1.

According to this aspect of the invention, the first outer coat having an integral structure is disposed in the soft part and the bending part. Therefore, it is not required to bond the outer coats together between the soft part and the bending part, and fluid-tightness ranging from the soft part to the bending part can be kept excellent by the first outer coat having an integral structure. Also, by using the second outer coat provided only to the soft part, the level of stiffness required for the soft part can be provided to the insertion part. Furthermore, by providing the second outer coat on the first outer coat, the position of the second outer coat with respect to the first outer coat can be easily defined with high accuracy.

Here, "the first outer coat having an integral structure" means elimination of a mode in which a plurality of portions are attached together by physical means, and the first outer coat can be configured of a single member or by combining a plurality of members.

Since the first outer coat has the same composition in the soft part and the bending part, the first outer coat having a simple structure provides excellent fluid-tightness.

A method of manufacturing the above endoscope is defined by claim 4.

The first outer coat may be coated with the second outer coat by inserting the first outer coat into the second outer coat having a sectional diameter larger than a sectional diameter of the first outer coat, and then reducing the second outer coat in a radial direction with the first outer coat inserted in the second outer coat.

According to this aspect of the invention, positioning and fixing the position of the second outer coat on the first outer coat can be easily performed with high accuracy. Note that the diameter of the second outer coat may be reduced with any method, and, for example, heat shrinkage or elastic shrinkage can be used.

The insertion part may further have a third outer coat with which at least part of the second outer coat is coated.

According to this aspect of the invention, the third outer coat can be arranged at a portion where further stiffness is required.

The first outer coat may have a large diameter part corresponding to the bending part and a small diameter part corresponding to the soft part and having a sectional diameter smaller than a sectional diameter of the large diameter part; and the second outer coat may have a thickness substantially equal to a difference between the sectional diameter of the large diameter part and the sectional diameter of the small diameter part, and the second outer coat with which the first outer coat is coated in the soft part may be arranged so as to be substantially flush with the first outer coat in the bending part.

According to this aspect of the invention, the outer circumference of the insertion part (the bending part and the soft part) has a flush and smooth surface, and therefore excellent insertability can be obtained.

According to the present invention, the integral first outer coat provided over the soft part and the bending part is used. Therefore, bonding of the outer coat between soft part and the bending part is not required, and excellent fluid-tightness ranging from the soft part to the bending part can be obtained with a simple structure. On the other hand, the level of stiffness required for the soft part can be easily ensured by the second outer coat provided only in the soft part. Also, the level of flexibility required for the bending part can be ensured by not forming the second outer coat in the soft part. Furthermore, since the second outer coat is provided on the first outer coat, the second outer coat can be easily positioned on the first outer coat with high accuracy.

### BRIEF DESCRIPTION OF THE DRAWINGS

Fig. 1 is an external view showing the entire structure of an endoscope;
Fig. 2 is an enlarged perspective view of a tip of an insertion part of the endoscope;
Fig. 3 is a sectional view of the insertion part of the endoscope, illustrating in particular a cross-section structure of a connecting portion between a soft part and a bending part;
Fig. 4A is a perspective view illustrating a step of forming an outer coat according to a first embodiment;
Fig. 4B is a perspective view illustrating a step of forming the outer coat according to the first embodiment;
Fig. 5 is an external view of the entire insertion part coated with the outer coat according to the first embodiment;
Fig. 6A is an external side view exemplarily illustrating a first outer coat layer configured of a plurality of materials, the first outer layer being formed of a plurality of materials on the soft part and the bending part;
Fig. 6B is an external side view exemplarily illustrating a first outer coat layer configured of a plurality of materials, with a material ratio in a transition section from the soft part to the bending part being gradually changed;
Fig. 6C is an external side view exemplarily illustrating a first outer coat layer configured of a plurality of materials, with different materials being mixed (blended) together in the transition section;
Fig. 6D is an external side view exemplarily showing a first outer coat layer configured of a plurality of materials, with a first material and a second material between which a third material is provided;
Fig. 7 is an external view of an entire insertion part coated with an outer coat according to a second embodiment when viewed from its side;
Fig. 8A is an external side view showing a step of forming an outer coat according to a third embodiment; and
Fig. 8B is an external side view of an entire insertion part coated with the outer coat according to the third embodiment when viewed from its side.

### DETAILED DESCRIPTION OF THE PREFERRED EMBODIMENTS

Embodiments of the present invention are described below with reference to the attached drawings. Note that for ease of understanding, devices are not necessarily rendered to scale in the respective drawings, but the relation among the respective devices can be duly understood from each of the drawings by persons skilled in the art. Also, the structures described below are merely examples, and the present invention can be applied also to an endoscope of another structure.

### Entire Structure of the Endoscope

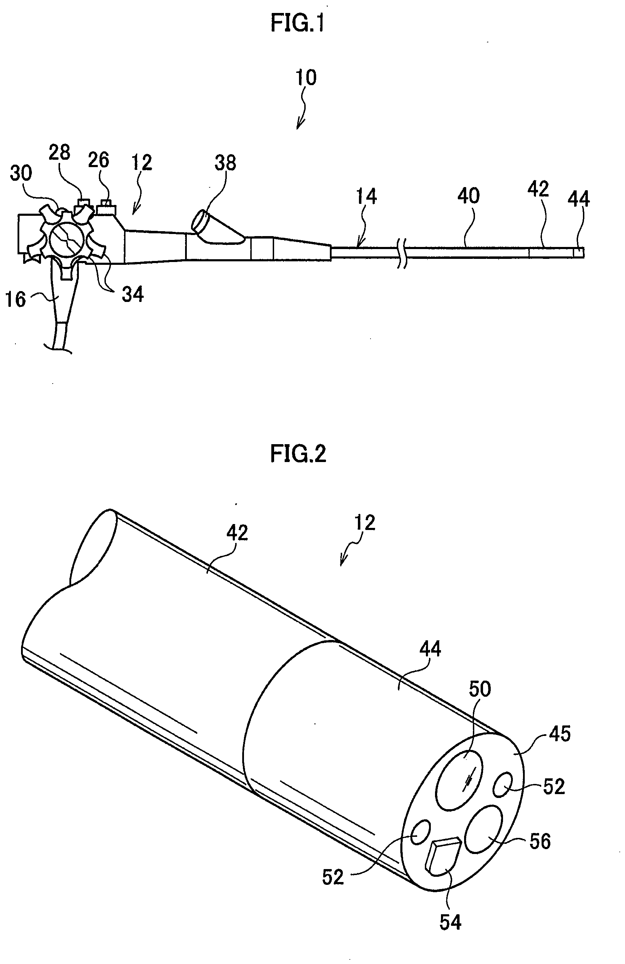

Fig. 1 is an external view showing the entire structure of an endoscope, and Fig. 2 is an enlarged perspective view of a tip of an insertion part of the endoscope.

As shown in Fig. 1, an endoscope 10 includes: a hand operating part 12 held by a user (an operator); and an insertion part 14 (a soft part 40, a bending part 42, and a tip hard part 44) connected to this hand operating part 12 and inserted into the body of a subject.

To the hand operating part 12, a universal cable 16 is connected. The universal cable 16 has its tip provided with a light guide connector (an LG connector) not shown. This LG connector is removably coupled to a light source device not shown. When the LG connector is connected to this light source device, illumination light can be sent to an illumination optical system 52 (refer to Fig. 2) disposed at the tip part (the tip hard part) 44 of the insertion part 14. Also, to the LG connector, an electric connector is connected via a cable, and the electric connector is removably coupled to a processor not shown. With the electric connector being connected to this processor, data of an observed image obtained by the endoscope 10 can be outputted to the processor, and furthermore, the observed image can be displayed on a monitor connected to the processor.

Also, the hand operating part 12 is provided thereon with an air/water supply button 26, a suction button 28, and a shutter button 30. The air/water supply button 26 is an operation button for ejecting air or water from an air/water nozzle 54 (refer to Fig. 2) disposed at the tip hard part 44 of the insertion part 14, causing air or water to be ejected from the air/water nozzle 54 toward an observation optical system (an observing lens) 50 provided at the tip hard part 44. Also, the suction button 28 is an operation button for suctioning a lesion, or the like, from a forceps port 56 (refer to Fig. 2) disposed at the tip hard part 44. The shutter button 30 is an operation button for operating recording and others of the observed image.

Furthermore, the hand operating part 12 is provided with paired angle knobs 34. By rotating the angle knobs 34, the user can remotely operate a bent state of the bending part (angle part) 42, thereby bending the bending part 42 in a desired direction.

Still further, the hand operating part 12 is provided with a forceps insertion part 38 which is connected to and communicated with the forceps port 56 of the tip hard part 44 (refer to Fig. 2). A treatment tool such as a clamp (forceps) is inserted from this forceps insertion part 38 and is led through a forceps channel (not shown) inside the soft part (flexible tube) 40 to go out from the forceps port 56.

On the other hand, the insertion part 14 is configured in such a manner that the soft part 40, the bending part 42, and the tip hard part 44 are disposed sequentially from the hand operating part 12 side. The soft part 40 is a flexible member formed in a cylindrical shape, and has a multilayered structure (such as an outer coat layer) that ensures required degrees of flexibility and stiffness, thereby playing a role of ensuring a route for the insertion part 14 during the time of insertion in the body. The bent state of the bending part 42 is controlled by the angle knobs 34 of the hand operating part 12 described above, in such a manner that the positions and directions of the observation optical system (observing lens) 50, the illumination optical system 52, the air/water nozzle 54, and the forceps port 56 provided at an end face 45 of the tip hard part 44 can be appropriately adjusted.

### Structure of the bending part and the soft part

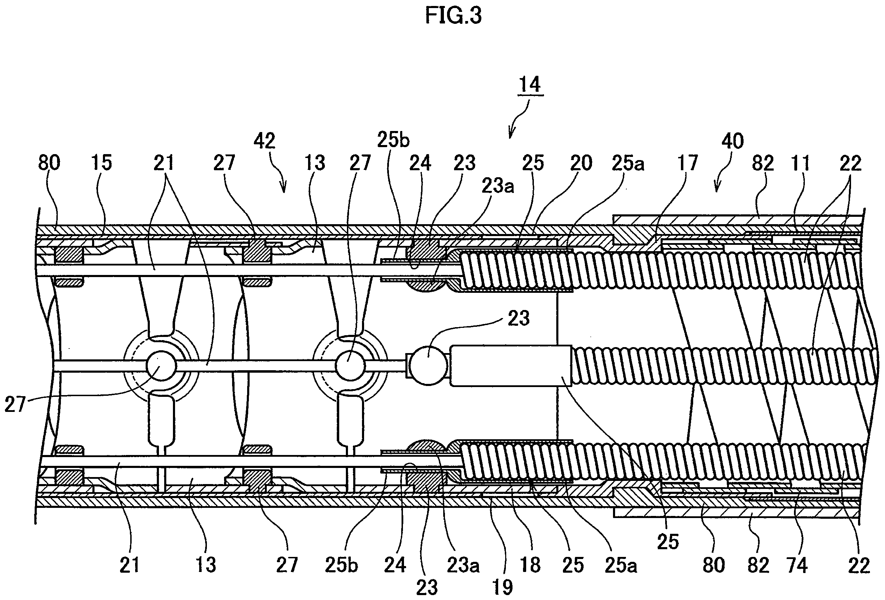

Fig. 3 is a sectional view of the insertion part 14 of the endoscope, showing in particular a cross-section structure of a connecting portion between the soft part 40 and the bending part 42.

The soft part 40 has a spiral tube (a flex) 74 formed of a metal band piece having a predetermined width wound in a spiral shape. The spiral tube 74 can be configured of, for example, doubled tubes with different wound directions. The spiral tube 74 is coated with a net (a net tube/braid) 11 formed of braided metal wires. This net 11 is coated with a first outer coat layer 80 and a second outer coat layer 82, which will be described further below.

On the other hand, the bending part 42 has a plurality of angle rings 13 with a joint ring structure mutually coupled to each other. The angle rings 13 are connected by paired pivotally-attaching pins 27 arranged at positions forming 180 degrees with respect to the axial direction of the insertion part 14. Each angle ring 13 can rotate in a direction orthogonal to the axis line of the pivotally-attaching pins 27. Coupling positions between the angle rings 13 with the pivotally-attaching pins 27 are changed horizontally or vertically with respect to the axial direction of the insertion part 14, and the bending part 42 can bend vertically and horizontally. The angle rings 13 joined together have their outer circumference coated with a net (net tube/braid) 15 formed of braided metal wires, like in the soft part 40. This net 15 is coated with the first outer coat layer 80, which will be described further below.

Since the soft part 40 and the bending part 42 have different internal structures, both are configured to be coupled together after portions other than the first outer coat layer 80 are separately manufactured. For coupling of these soft part 40 and bending part 42, the soft part 40 has its tip part provided with a soft-part-side coupling ring 17, and the bending part 42 has its base end provided with a bend-part-side coupling ring 18. With these coupling rings 17 and 18 fitting in each other, the soft part 40 and the bending part 42 are coupled together. The soft-part-side coupling ring 17 is fixedly attached by soldering, welding, or the like, to the tip of the spiral tube 74 of the soft part 40, and the bend-part-side coupling ring 18 is coupled to the angle ring 13 on a base endmost side by predetermined fixing means (for example, means such as the pivotally-attaching pins 27, soldering, and welding). The bend-part-side coupling ring 18 partially fits by insertion in the soft-part-side coupling ring 17, and solder 20 is poured into a through hole 19 formed in the soft-part-side coupling ring 17 positioned outside, thereby coupling the soft-part-side coupling ring 17 and the bend-part-side coupling ring 18 together.

Note that a plurality of operation wires 21 are provided inside the insertion part 14 (the soft part 40 and the bending part 42) for bending the bending part 42 with a remote operation, and the operation wires 21 are operated with the angle knobs 34 provided in the hand operating part 12 in an interlocked manner. When the bending part 42 is bent in a vertical direction and a horizontal direction, four operation wires 21 are provided at four positions above, below, at left, and at right with respect to the axial direction of the insertion part 14 (at positions at 90 degrees with respect to the axial direction of the insertion part 14). That is, the operation wires 21 are formed of a vertical pair and a horizontal pair, and when the angle knobs 34 are operated so that one of the paired vertical operation wires 21 is drawn to the hand operating part 12 side and the other is unreeled, the bending part 42 is bent in a vertical direction. Also, when the angle knobs 34 are operated so that one of the paired horizontal operation wires 21 is drawn to the hand operating part 12 side and the other is paid out, the bending part 42 is bent in a horizontal direction. Note that the paired operation wires 21 are not necessarily provided above, below, at left, and at right and, for example, the structure can be such that the paired operation wires 21 are provided above and below only. The operation wires 21 as described above each have its tip fixedly attached to the foremost angle ring 13 of the bent part 42 or the tip hard part 44 (refer to Fig. 1). In the bending part 42, the operation wires 21 are each inserted through an insertion hole formed in the relevant pivotally-attaching pin 27. In the soft part 40, the operation wires 21 are each inserted through a close-contact coil 22 as a flexible guide tube. With this structure, the position of each operation wire 21 with respect to the circumferential direction of the insertion part 14 is kept.

Note that the tip of a close-contact coil 22 functioning as guide means for each operation wire 21 is fixed to a predetermined portion of a coupling part between the soft part 40 and the bending part 42. More specifically, tying-up pins 23 are provided with the bend-part-side coupling ring 18 to be tied up thereto by means such as caulking, and a head part 23a of each tying-up pin 23 positioned inside the bend-part-side coupling ring 18 has an insertion hole 24 formed therein. To this insertion hole 24, a fixing pipe 25 for fixing the tip of each close-contact coil 22 is fixedly attached, as being inserted, by means such as brazing. The fixing pipe 25 has a large diameter part 25a positioned on a soft part 40 side and a small diameter part 25b positioned on a bending part 42 side, and the large diameter part 25a and the small diameter part 25b form a step. The tip of each close-contact coil 22 having the operation wire 21 inserted therethrough is fixedly attached, as being inserted, to the large diameter part 25a by means such as laser spot welding. On the other hand, only the operation wire 21 is inserted through the small diameter part 25b inserted through the insertion hole 24 of the tying-up pin 23.

In addition, although not shown in the drawings, a forceps channel connecting to the forceps insertion part 38 shown in Fig. 1 and the forceps port 56 shown in Fig. 2 and causing the forceps insertion part 38 and the forceps port 56 to communicate with each other, a signal line, a light-guiding line guiding light to the illumination optical system 52, an air/water line communicating with the air/water nozzle 54, and others are appropriately provided as required inside the soft part 40 and the bending part 42.

After the soft part 40 and the bending part 42 coupled together in this manner, the internal structure of the soft part 40 and the bending part 42 is coated with the outer coat (the first outer coat layer 80 and the second outer coat layer 82).

### Structure of outer coat

Next, embodiments of the outer coat configuring an outer circumferential portion of the insertion part 14 (in particular, the soft part 40 and the bending part 42) are described. Note that while feature portions of the outer coat are highlighted in each drawing for ease of understanding, the specific size, shape, and others of each embodiment are determined as appropriated.

### First embodiment

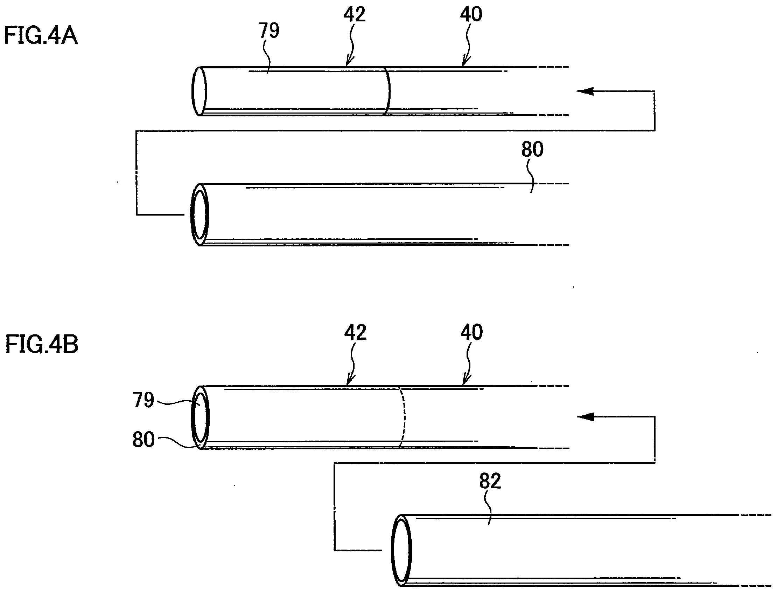

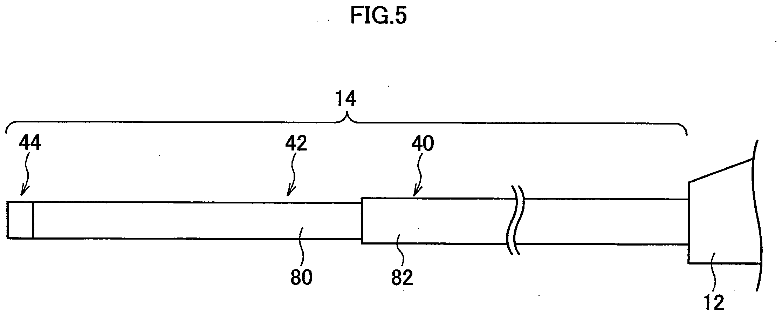

Fig. 4A and Fig. 4B are perspective views showing steps of forming an outer coat according to a first embodiment. Note in Fig. 4A and Fig. 4B that while the internal structure of the soft part 40 and the bending part 42 other than the outer coat (refer to Fig. 3) is illustrated as a cylindrical member 79. Also, Fig. 5 is an external view of the entire insertion part 14 coated with the outer coat according to the first embodiment.

The outer coat according to the present embodiment is configured of a first outer coat layer 80 provided over the soft part 40 and the bending part 42 and a second outer coat layer 82 covering only the soft part 40. More specifically, first as shown in Fig. 4A, the internal structure 79 of the soft part 40 and the bending part 42 is coated with the first outer coat layer 80. This first outer coat layer 80 has an integral structure over all, coating the entire soft part 40 and bending part 42 with a uniform surface property without including a connection structure. Then, as shown in Fig. 4B, the first outer coat layer 80 is coated with the second outer coat layer 82 so that the second outer coat layer 82 covers only the soft part 40.

In the insertion part 14 thus coated with the first outer coat layer 80 and the second outer coat layer 82, the entire soft part 40 and bending part 42 is coated with the first outer coat layer 80 having an integral structure without a bonding part, as shown in Fig. 5. Therefore, the insertion part 14 according to the present embodiment has excellent fluid-tightness (air-tightness) over a long period of time.

Also, while the bending part 42 is coated with only the first outer coat layer 80, the first outer coat layer 80 of the soft part 40 is coated with the second outer coat layer 82. Therefore, an appropriate degree of stiffness (flexibility) can be provided to each of the bending part 42 and the soft part 40 in a simple manner. More specifically, while the material, thickness (material thickness), and others of the first outer coat layer 80 are determined in view of ensuring flexibility not inhibiting a bending operation of the bending part 42, the material, thickness, and others of the second outer coat layer 82 are determined in view of ensuring a stiffness necessary for the soft part 40, thereby allowing different degrees of stiffness (flexibility) to be provided to the bending part 42 and the soft part 40.

Note that the method of fixing the first outer coat layer 80 and the second outer coat layer 82 is not particularly restricted, and with a fixing means such as an adhesive to be given to a predetermined position, the first outer coat layer 80 and the second outer coat layer 82 can be fixed for coating.

Also, with the first outer coat layer 80 inserted into (inside of) the second outer coat layer 82, a fastening force (a friction force) of the second outer coat layer 82 occurring by reducing the diameter of the second outer coat layer 82 to narrow the sectional diameter can be used to accurately fix a relative position of the second outer coat layer 82 with respect to the first outer coat layer 80. For example, in the case in which the second outer coat layer 82 is formed of an elastic member, when the first outer coat layer 80 is inserted inside the second outer coat layer 82, the sectional diameter of the second outer coat layer 82 is elastically enlarged, and the first outer coat layer 80 is inserted into the second outer coat layer 82 with its sectional diameter larger than that of the first outer coat layer 80. Then, with the second outer coat layer 82 fitting in the outside of the first outer coat layer 80, the sectional diameter of the second outer coat layer 82 is elastically reduced, thereby allowing the first outer coat layer 80 to be coated with the second outer coat layer 82 in a close contact manner. Also, in the case in which the second outer coat layer 82 is configured of a heat-shrinkable member, when the first outer coat layer 80 is inserted inside the second outer coat layer 82, the second outer coat layer 82 having a sectional diameter larger than that of the first outer coat layer 80 is used, and with the second outer coat layer 82 fitting outside the first outer coat layer 80, the second outer coat layer 82 is heated to reduce the sectional diameter of the second outer coat layer 82, thereby allowing the first outer coat layer 80 to be coated with the second outer coat layer 82 in a close contact manner.

Also, the material configuring the first outer coat layer 80 and the second outer coat layer 82 each having an integral structure is not particularly restricted, and any material satisfying functions and characteristics required for each layer (such as flexibility, waterproofness, durability, surface smoothness, chemical resistance, autoclave resistance, washability, or cleaning resistance) can be used to configure the first outer coat layer 80 and the second outer coat layer 82. For example, while the first outer coat layer 80 with which both of the soft part 40 and the bending part 42 are coated may be formed from a fluoro-rubber tube, the second outer coat layer 82 with which only the soft part 40 is coated may be formed from a fluoro-resin tube or an urethane resin tube.

Furthermore, the first outer coat layer 80 and the second outer coat layer 82 having an integral structure may be configured of a single material of the same composition, or may be configured by combining a plurality of materials having different compositions.

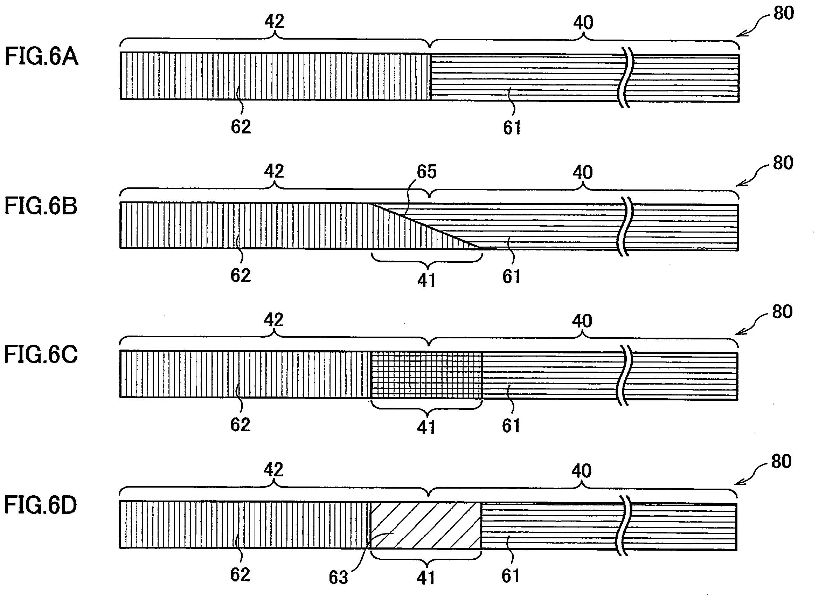

Fig. 6A to Fig. 6D are external side views exemplarily showing the first outer coat layer 80 configured of a plurality of materials, with a difference in material being represented by a difference in hatching in the drawings.

For example, as shown in Fig. 6A, the first outer coat layer 80 of the soft part 40 may be formed of a first material 61, and the first outer coat layer 80 of the bending part 42 may be formed of a second material 62. Also, as shown in Fig. 6B, in a transition section 41 of a predetermined range of the first outer coat layer 80 from the soft part 40 to the bending part 42, the first outer coat layer 80 may be formed of the first material 61 and the second material 62 with their ratio successively changed so that an interface 65 between the first material 61 and the second material 62 is flat.

Also, as shown in Fig. 6C, the transition section 41 of the first outer coat layer 80 described above may be configured of a composition in a blended state with the first material 61 and the second material 62 mixed together. In this case, the blending ratio between the first material 61 and the second material 62 in the transition section 41 can be changed in a manner such that the blending ratio of the first material 61 on the soft part 40 side is higher than that of the second material 62 and the blending ratio of the second material 62 on the bending part 42 side is higher than that of the first material 61. The blending ratio may be successively changed from the soft part 40 to the bending part 42.

Furthermore, as shown in Fig. 6D, in the transition section 41 described above, the first outer coat layer 80 may be configured of a third material (third composition part) 63 different from the first material (first composition part) 61 and the second material (second composition part) 62. In view of integrally forming the first outer coat layer 80, this third material 63 is preferably excellent in bonding with an affinity for both of the first material 61 and the second material 62.

Note that the first material 61, the second material 62, and the third material 63 described above are not particularly restricted, and any material capable of configuring the integral first outer coat layer 80 can be selected. Therefore, for example, a urethane-based elastomer or an ester-based elastomer may be used as the first material 61, the second material 62, or the third material 63, and also, the hardness can be varied for each material.

### Second Embodiment

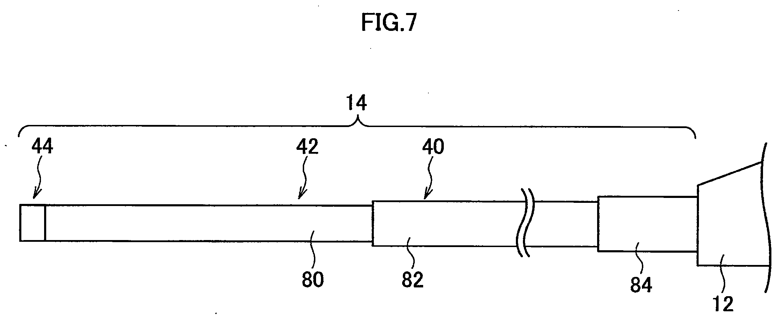

Fig. 7 is an external view of the entire insertion part 14 coated with an outer coat according to a second embodiment when viewed from its side.

The insertion part 14 of the present embodiment has a third outer coat layer 84 with which the second outer coat layer 82 is coated. This third outer coat layer 84 is provided in a predetermined range on the base end of the second outer coat layer 82 (an end on the hand operating part 12 side).

Other components each have a structure similar to that of the outer coat according to the first embodiment described above.

With this third outer coat layer 84 disposed, the stiffness and strength of the root portion (the end on the hand operating part 12 side) of the soft part 40 can further be reinforced, thereby further improving operation performance of the insertion part 14.

Note that the third outer coat layer 84 can be used for coating and fixed in a manner similar to that of the second outer coat layer 82, and can be configured of any material (for example, a fluoro-resin tube).

### Third Embodiment

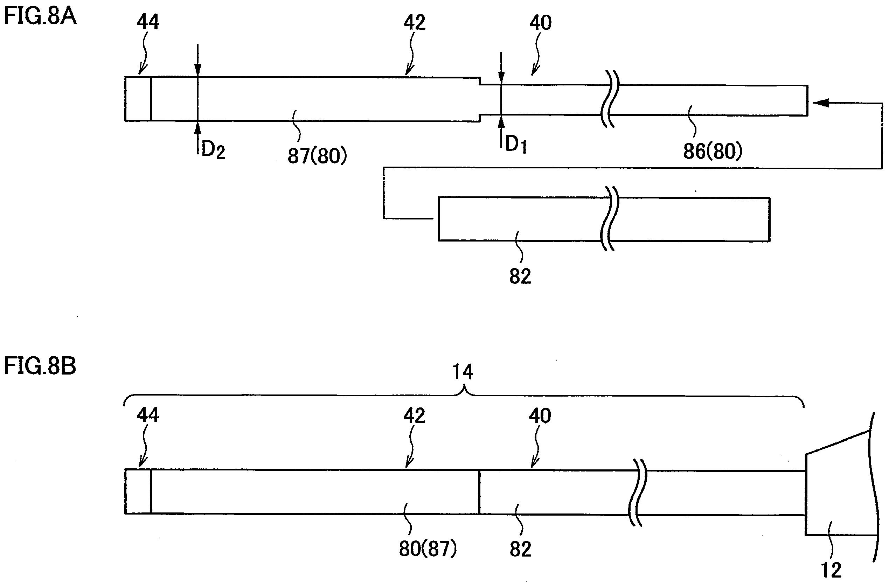

Fig. 8A is an external side view showing steps of forming an outer coat according to a third embodiment, and Fig. 8B is an external side view of the entire insertion part 14 coated with the outer coat according to the third embodiment when viewed from its side.

The first outer coat layer 80 of the present embodiment has a small diameter part 86 corresponding to the soft part 40 and a large diameter part 87 corresponding to the bending part 42, with the small diameter part 86 and the large diameter part 87 being successively and integrally formed. The small diameter part 86 has an outer diameter D₁ smaller than an outer diameter D₂ of the large diameter part 87, and a difference between the outer diameter D₂ of the large diameter part 87 and the outer diameter D₁ of the small diameter part 86 is approximately equal to the thickness of the second outer coat layer 82 (thickness in a sectional diameter direction) (D₂ - D₁ ≈ thickness of the second outer coat layer 82).

Therefore, as shown in Fig. 8A, the first outer coat layer 80 has a step part formed between the small diameter part 86 (the soft part 40) and the large diameter part 87 (the bending part 42), and with the tip part of the second outer coat layer 82 (an end on the tip hard part 44 side) abutting on this step part, the small diameter part 86 is coated with the second outer coat layer 82.

Other components each have a structure similar to that of the outer coat according to the first embodiment described above.

According to the present embodiment, since difference between the outer diameter D₂ of the large diameter part 87 and the outer diameter D₁ of the small diameter part 86 of the first outer coat layer 80 corresponds to the thickness of the second outer coat layer 82, then when the first outer coat layer 80 (the small diameter part 86) is coated with the second outer coat layer 82, as shown in Fig. 8B, an outer-coat outer circumferential surface of the bending part 42 (the large diameter part 87 of the first outer coat layer 80) and an outer-coat outer circumferential surface of the soft part 40 (the second outer coat layer 82) are arranged so as to be flush with each other. Therefore, according to the present embodiment, the outer circumferential surface of the insertion part 14 (in particular, a portion between the soft part 40 and the bending part 42) can be formed to be smooth, and more excellent insertability can be provided to the insertion part 14.

While the outer coat structures of various embodiments have been exemplarily shown above with reference to Fig. 4A to Fig. 8B, the present invention is not limited to these illustrated examples.

For example, while the example has been described in the above-described embodiments in which the first outer coat layer 80 is provided over the entire soft part 40 in the embodiments described above, the first outer coat layer 80 may be formed so as to cover at least part of the soft part 40 and the bending part 42. More specifically, fluid-tightness between the bending part 42 and the soft part 40 can be excellently kept as long as at least part of the soft part 40 and the bending part 42 are coated with the first outer coat layer 80 having an integral structure. In this case, since the soft part 40 is covered with the second outer coat layer 82, a region coated with the first outer coat layer 80 and the second outer coat layer 82 and a region coated only with the second outer coat layer 82 are present in the soft part 40.

Also, while an example has been described in the above-described embodiments in which the first outer coat layer 80 is provided over the soft part 40 and the bending part 42, the first outer coat layer 80 can be not only provided to the soft part 40 and the bending part 42 but also be provided so as to form an outermost layer of the tip hard part 44.

While preferred embodiments of the present invention have been described, the present invention is not limited to the embodiments described above, and can be applied as appropriate to another embodiment within the scope of the appended claims.

## Claims

1. An endoscope (10) comprising an insertion part (14) including a bending part (42) bending with a remote operation and a soft part (40) connected to the bending part (42), wherein
the insertion part (14) has: a first outer coat (80) with an integral structure provided over the entire soft part (40) and in the bending part (42); and a second outer coat (82) with which the first outer coat (80) is coated in the soft part (40) only, and the first outer coat (80) has a same composition in the soft part (40) and the bending part (42),
**characterized in that** the bending part (42) is coated only by the first outer coat (80).

2. The endoscope (10) according to claim 1, wherein the insertion part (14) further has a third outer coat (84) with which the second outer coat (82) is coated.

3. The endoscope (10) according to claim 1 or 2, wherein:
the first outer coat (80) has a large diameter part (87) corresponding to the bending part (42) and a small diameter part (86) corresponding to the soft part (40) and having a sectional diameter smaller than a sectional diameter of the large diameter part (87); and
the second outer coat (82) has a thickness substantially equal to a difference between the sectional diameter of the large diameter part (87) and the sectional diameter of the small diameter part (86), and the second outer coat (82) with which the first outer coat (80) is coated in the soft part (40) is arranged so as to be substantially flush with the first outer coat (80) in the bending part (42).

4. A method of manufacturing an endoscope according to claim 1, comprising the steps of coating the first outer coat (80), which is provided over the entire soft part (40) and in the bending part (42), with the second outer coat (82) in the soft part only, by inserting the first outer coat (80) into the second outer coat (82) having a sectional diameter larger than a sectional diameter of the first outer coat (80), and then reducing the second outer coat (82) in a radial direction with the first outer coat (80) inserted in the second outer coat (82).

## Patentansprüche

1. Endoskop (10), umfassend ein Einführteil (14), das ein durch Fernbetätigung biegbares Biegeteil (42) und ein mit dem Biegeteil (42) verbundenes weiches Teil (40) enthält, wobei
das Einführteil (14) aufweist: eine erste Außenumhüllung (80) mit einer integralen Struktur über dem gesamten weichen Teil (40) und in dem Biegeteil (42); und eine zweite Außenumhüllung (42), mit der die erste Außenumhüllung (80) innerhalb ausschließlich des weichen Teils (40) umhüllt ist, und die erste Außenumhüllung (80) in dem weichen Teil (40) und dem Biegeteil (42) die gleiche Zusammensetzung aufweist,
**dadurch gekennzeichnet, dass** das Biegeteil (42) nur von der ersten Außenumhüllung (80) umhüllt ist.

2. Endoskop (10) nach Anspruch 1, bei dem das Einführteil (14) weiterhin eine dritte Außenumhüllung (84) aufweist, mit welcher die zweite Außenumhüllung (82) umhüllt ist.

3. Endoskop (10) nach Anspruch 1 oder 2, bei dem:
die erste Außenumhüllung (40) einen Abschnitt großen Durchmessers (87) entsprechend dem Biegeteil (42) und einen Abschnitt kleinen Durchmessers (86) entsprechend dem weichen Teil (40) und mit einem Querschnittsdurchmesser kleiner als ein Querschnittsdurchmesser des Teils (87) großen Durchmessers aufweist; und
die zweite Außenumhüllung (82) eine Dicke aufweist, die im wesentlichen einer Differenz zwischen dem Querschnittsdurchmesser des Teils (87) großen Durchmessers und dem Querschnittsdurchmesser des Teils (86) kleinen Durchmessers gleicht, und die zweite Außenumhüllung (82), mit der die erste Außenumhüllung (80) in dem weichen Teil (40) umhüllt ist, derart angeordnet ist, dass sie in dem Biegeteil (42) im wesentlichen bündig ist mit der ersten Außenumhüllung (80).

4. Verfahren zum Fertigen eines Endoskops nach Anspruch 1, umfassend die Schritte des Umhüllens der ersten Außenumhüllung (80), die über dem gesamten weichen Teil (40) und in dem Biegeteil (82) vorgesehen ist, mit der zweiten Außenumhüllung (82) lediglich in dem weichen Teil, indem die erste Außenumhüllung (80) in die zweite Außenumhüllung (82) eingeführt wird, die einen größeren Querschnittsdurchmesser aufweist als die erste Außenumhüllung (80), und anschließend die zweite Außenumhüllung (82) in radialer Richtung verkleinert wird, während die erste Außenumhüllung (80) in die zweite Außenumhüllung (82) eingeführt ist.

## Revendications

1. Endoscope (10), comprenant une partie d'introduction (14) incluant une partie de flexion (42) fléchissant avec une manoeuvre à distance et une partie souple (40) reliée à la partie de flexion (42),
dans lequel
la partie d'introduction (14) présente : un premier revêtement extérieur (80) présentant une structure intégrale prévue sur toute la partie souple (40) et dans la partie de flexion (42), et un deuxième revêtement extérieur (82) avec lequel le premier revêtement extérieur (80) est recouvert uniquement dans la partie souple (40), et le premier revêtement extérieur (80) présente une composition identique dans la partie souple (40) et la partie de flexion (42),
**caractérisé en ce que** la partie de flexion (42) est recouverte uniquement par le premier revêtement extérieur (80).

2. Endoscope (10) selon la revendication 1, dans lequel la partie d'introduction (14) présente en outre un troisième revêtement extérieur (84) avec lequel le second revêtement extérieur (82) est recouvert.

3. Endoscope (10) selon la revendication 1 ou 2, dans lequel :
le premier revêtement extérieur (80) présente une partie de grand diamètre (87) correspondant à la partie de flexion (42) et une partie de petit diamètre (86) correspondant à la partie souple (40) et présentant un diamètre de section inférieur à un diamètre de section de la partie de grand diamètre (87), et
le second revêtement extérieur (82) présente une épaisseur en grande partie égale à une différence entre le diamètre de section de la partie de grand diamètre (87) et le diamètre de section de la partie de petit diamètre (86), et le deuxième revêtement extérieur (82) avec lequel le premier revêtement extérieur (80) est recouvert dans la partie souple (40) est agencé de manière à se trouver en grande partie à ras avec le premier revêtement extérieur (80) dans la partie de flexion (42).

4. Procédé de fabrication d'un endoscope selon la revendication 1, comprenant les étapes consistant à recouvrir le premier revêtement extérieur (80), lequel est prévu sur toute la partie souple (40) et dans la partie de flexion (42), avec le deuxième revêtement extérieur (82) dans la partie souple (40) uniquement, en introduisant le premier revêtement extérieur (80) dans le second revêtement extérieur (82) présentant un diamètre de section supérieur à un diamètre de section du premier revêtement extérieur (80), puis en réduisant le second revêtement extérieur (82) dans une direction radiale, le premier revêtement extérieur (80) étant introduit dans le deuxième revêtement extérieur (82).
